(19) Europäisches Patentamt
European Patent Office
Office européen des brevets

(11) **EP 4 553 174 A1**

(12) **EUROPEAN PATENT APPLICATION**

(43) Date of publication:
**14.05.2025 Bulletin 2025/20**

(21) Application number: **23383145.2**

(22) Date of filing: **08.11.2023**

(51) International Patent Classification (IPC):
***C12Q 1/6883*** (2018.01)

(52) Cooperative Patent Classification (CPC):
(C-Sets available)
**C12Q 1/6883;** C12Q 1/686; C12Q 2600/158
(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Consejo Superior De Investigaciones Científicas**
**28006 Madrid (ES)**
• **Servicio Andaluz de Salud**
**41071 Sevilla (ES)**

(72) Inventors:
• **RUIZ-MATEOS CARMONA, Ezequiel**
**41013 Sevilla (ES)**
• **GASCA CAPOTE, María Carmen**
**41013 Sevilla (ES)**

(74) Representative: **Balder IP Law, S.L.**
**Paseo de la Castellana 93**
**5ª planta**
**28046 Madrid (ES)**

Remarks:
The complete document including Reference
Table(s) and the Sequence Listing(s) can be
downloaded from the EPO website

(54) **METHODS TO DETERMINE THE THYMIC FUNCTION BY DROPLET DIGITAL PCR SYSTEM**

(57)    The invention disclosed herein provides an *in vitro* and/or *ex vivo* method to assess and monitoring thymic function and also an *in vitro* and/or *ex vivo* method for prognosis and/or diagnosis of immune system dysfunction in an isolated biological sample from an individual comprising the detection of the sj/β-TREC (T-cell receptor Rearrangement Excision Circles) ratio by droplet digital PCR (ddPCR). Moreover, the present invention also relates to the *in vitro* and/or *ex vivo* use of the sj/DβJβ-TREC ratio as a biomarker for the assess and monitoring thymic function and methods for the prognosis and/or diagnosis of immune system dysfunction in an isolated sample of an individual by a ddPCR. In addition, the present invention also refers to a kit for use in these methods.

(52)  Cooperative Patent Classification (CPC): (Cont.)

C-Sets
C12Q 1/686, C12Q 2563/159

Description

## TECHNICAL FIELD

The invention disclosed herein provides an *in vitro* and/or ex *vivo* method to assess and monitor thymic function and also an *in vitro* and/or ex *vivo* method for prognosis and/or diagnosis of immune system dysfunction in an isolated biological sample from an individual comprising the detection of the sj/DβJβ-TREC (T-cell receptor Rearrangement Excision Circles) ratio by droplet digital PCR (ddPCR). The methods disclosed herein allow the absolute quantification (copies per cell) of both regions. Moreover, the present invention also relates to the *in vitro* and/or ex *vivo* use of the sj/DβJβ-TREC ratio as a biomarker for the assessment and monitorization of thymic function and methods for the prognosis and/or diagnosis of immune system dysfunction in an isolated sample of an individual by a ddPCR. In addition, the present invention also refers to a kit for use in these methods.

## STATE OF THE ART

The thymus is one of the main organs involved in the generation and maintenance of the immune system, being essential for T-cell development, T-cell reconstitution after haematopoietic stem-cell transplantation, intensive chemotherapy, or HIV infection. During aging, the thymus undergoes a gradual atrophy process leading to a progressive decline in its function, known as thymic involution. This process is characterized by a gradual decrease in the supply of new T-lymphocytes to the peripheral blood, the main trigger for immunosenescence. A significant decrease in naïve T-cell production reduces the diversity of the T-cell antigen receptor (TCR) repertoire, modifying T-cell homeostasis. In addition to a deficient naive T-cells production, immunosenescence is also linked to an amplified oligoclonal expansion of memory T-cells. Thus, determining the functionality of the thymus and its role in maintaining the peripheral population of naive lymphocytes and inflammation, is necessary for understanding the process of immunosenescence. As thymic biopsies are an invasive and contraindicated technique, indirect methods for measuring thymic output in peripheral blood have been used in the prior art (Ferrando-Martinez S, et al.; J Immunol Methods. 2010;352(1-2):111-117; Dion M-L, et al. Methods Mol Biol. 2007:197-213).

Thymic output can be measured by assessing the presence of T-cell receptor rearrangement excision circles (TRECs) in naive T-cells, indicators of recent thymic emigrants in humans. TRECs are small episomal circles of DNA that are created on T-cells during their passage through the thymus when they rearrange their T-cells receptor (TCR) genes since TRECs are not replicated during T-cell proliferation in the periphery. TRECs content of T-cell populations correlates with thymic output; therefore, in aging, the presence of TRECs decreases as part of the normal thymic ageing involution process. Although the measurement of the signal joint (sj-TRECs) is widely used to measure the thymic function, this is not an accurate measurement since it is affected by peripheral proliferation mechanisms (Harris JM, et al., Clin Immunol. 2005; 115(2): 138-146; Hazenberg MD, et al., Nat Med. 2000;6(9):1036-1042). In certain clinical conditions, such as bone marrow transplantation or HIV infection, the increased proliferation of naive T-cells, reduces the frequency of TREC molecules which entails the loss of the relationship between sj-TREC frequency and the thymic output (Dion M-L, et al., Methods Mol Biol. 2007:197-213).

To solve this issue, two different types of TRECs are usually quantified: (i) the six DβJβ-TRECs, included in cluster 1, a product of the β chain TCR rearrangement at the most immature thymocyte subset; and (ii) the sj-TREC, a product of the α chain TCR rearrangement. The sj/DβJβ-TREC ratio quantification represents an intra-thymic proliferation step, that reflects thymus functionality and is not altered by proliferation in the periphery (Dion M-L, et al., Methods Mol Biol. 2007:197-213; den Braber I, et al., Immunity. 2012;36(2):288-297; Dion M-L, et al., Immunity. 2004;21:757-768).

Originally, the technique based on quantitative real-time PCR (qPCR), was excessively tedious, time-consuming, and required large amounts of sample. In order to overcome the restrictions existing in the prior art for the sj/DβJβ-TREC ratio quantification by qPCR, a new simplified method of this technique by reducing the number of PCR reactions from 33 to 9 was disclosed (Ferrando-Martinez S, et al. J Immunol Methods. 2010;352(1-2):111-117). Although promising, this new simplified method and results obtained therefrom cannot be considered efficient since it still required many PCR reactions, including the independent amplification of both regions by conventional PCR before the qPCR, and the generation of a standard curve.

There is still a need for new methods that overcome all the above limitations.

## DESCRIPTION OF THE INVENTION

The present disclosure reveals new improved methods by ddPCR for the sj/DβJβ-TREC ratio quantification that overcomes all the above limitations, by the use of specific primers and probes.

The methods of the present disclosure allow the absolute quantification in a single test, without the need for a standard curve of two different types of TRECs: DβJβ-TRECs and sj-TREC, expressed as copies per cell. The absolute quantification of the DβJβ-TRECs and sj-TREC is performed by a set of primers and probes disclosed herein, each of which has a sequence of nucleotides that is complementary with the sequences deposited in the GenBank database (NCBI) with accession number No. L36092.2 and in SEQ ID NO: 31 for DβJβ-TRECs, or with the sequence deposited in the GenBank database (NCBI) with accession number No. AE000521.1 and in SEQ ID NO: 32 for sj-TREC.

Thus, the primers and probes for measuring the expression level of sj-TREC by ddPCR are set for as SEQ ID NO: 10, 11 and 26, respectively and the primers and probes used for measuring the expression level of DβJβ-TREC by ddPCR, are set forth as SEQ ID NO: 13, 20 to 25 and 27, respectively. In a preferred embodiment, the probes of the present disclosure comprise a detectable moiety and/or a quencher.

The methods of the present disclosure are faster than the previous methods known in the art since it does not need multiple PCR reactions and present higher sensitivity, accuracy, and reproducibility than those method known in the art. Thus, the present disclosure refers to the use of the sj/DβJβ-TREC ratio analyzed by ddPCR in an isolated biological sample of an individual, as a marker for evaluating thymic function, which has the advantages above-mentioned. The latter can be useful in the prognosis and/or diagnosis of viral infection, vaccination, antiviral therapy and/or immune status (namely in HIV patients), graft, autoimmune disease, etc.

Droplet Digital PCR technology is a tool for quantitative measurement of absolute DNA concentration. As opposed to the relative measurement obtained from the real-time PCR reactions, ddPCR enables absolute measurement of target deoxyribonucleic acid (DNA), ribonucleic acid (RNA) molecules without the need for a standard curve.

Absolute quantification is advantageous in several applications, such as measuring copy number variation, detecting rare sequences and mutations, and analyzing gene expression reactions. ddPCR technology is a digital PCR method utilizing a water-oil emulsion droplet system. Droplets are formed in a water-oil emulsion to form the partitions that separate the template DNA molecules. The droplets serve essentially the same function as individual test tubes or wells in a plate in which the PCR reaction takes place, albeit in a much smaller format. The massive sample partitioning is a key aspect of the ddPCR technique. The ddPCR System partitions nucleic acid samples into thousands of nanoliter-sized droplets, and PCR amplification is carried out within each droplet. This technique has a smaller sample requirement than other commercially available digital PCR systems, reducing cost and preserving precious samples. Sample partitioning is the key to ddPCR. In traditional PCR, a single sample offers only a single measurement, but in ddPCR, the sample is partitioned into 20,000 nanoliter-sized droplets. This partitioning enables the measurement of thousands of independent amplification events within a single sample. Exemplary compositions for carrying out dPCR can include template nucleic acid (e.g., isolated DNA from cells known or suspected of containing a reverse transcribed viral nucleic acid), fluorescence-quencher probes, primers, and a PCR master mix, which contains DNA polymerase, dNTPs, MgCh, and reaction buffers at optimal concentrations. The PCR solution is divided into smaller reactions and is then made to run PCR individually. The partitioning of the sample allows one to estimate the number of different molecules by assuming that the molecule population follows the Poisson distribution, thus accounting for the possibility of multiple target molecules inhabiting a single partition._Exemplary commercially available apparatuses or systems for dPCR include Raindrop™ Digital PCR System (Raindance™ Technologies); QX200™ Droplet Digital™ PCR System (Bio-Rad); BioMark™ HD System and qdPCR 37K™ IFC (Fluidigm Corporation) and QuantStudio™ 3D Digital PCR System (Fife Technologies™) (see, e.g., Huggett et al. (2013) Clinical Chemistry 59: 1691-1693; Shuga, et al. (2013) Nucleic Acids Research 41(16): el59; Whale et al. (2013) PLoS One 3: e58177).

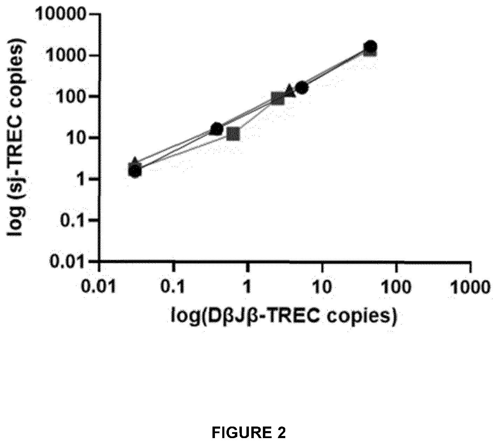

First, the inventors determined the detection limit for the ddPCR as < 2 copies/μl, which demonstrated that ddPCR determination is a reliable and accurate method to use for the determination of thymic function (see **Example 1 and Figure 2**).

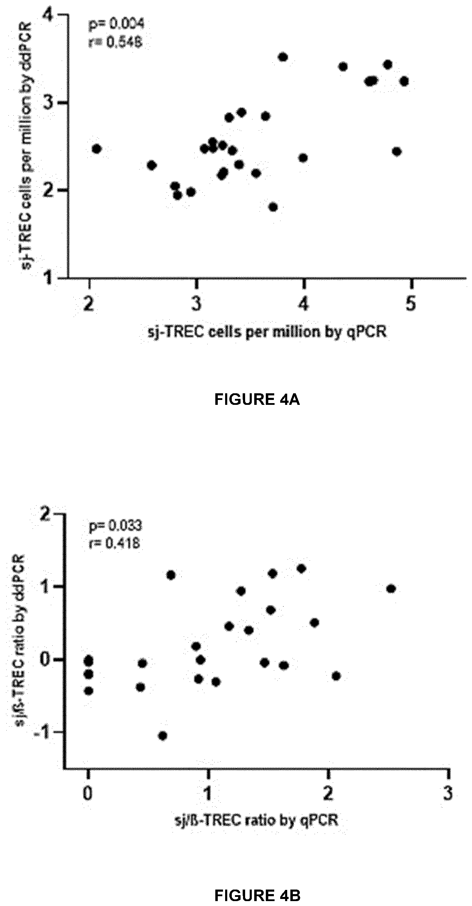

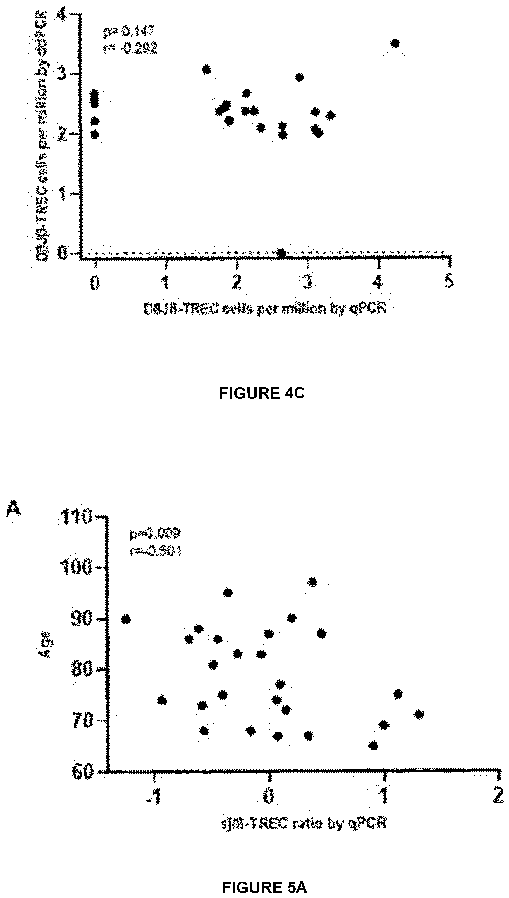

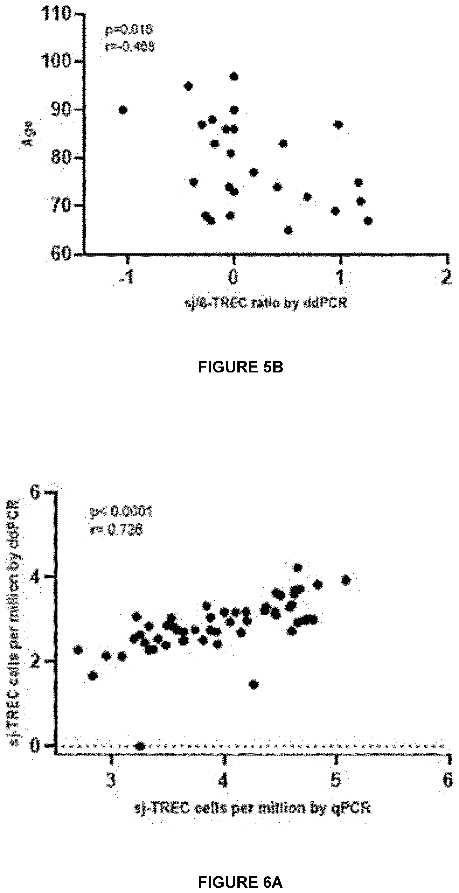

Then, it was compared the thymic function parameters (DβJβ-TRECs and sj-TREC) using the two methods, ddPCR and qPCR, in two different populations where thymic function failure has been already described, elderly healthy donors and people living with HIV. Thymic function persists in adulthood up to 107 years of age and failures in this function are associated with mortality in the elderly population. In the present disclosure, a positive correlation for sj-TREC and sj/DβJβ-TREC ratio in elderly healthy donors, measured by ddPCR and qPCR was demonstrated (see **Example 2 and Figure 4**). However, no differences were observed for DβJβ-TRECs, which may be due to the 28% of the measurements were below the detection limit using the qPCR approach (See **Example 2 and Figure 5**). This finding highlights the higher sensitivity and precision of ddPCR to detect the DβJβ-TRECs in comparison with conventional qPCR. Although the TREC level undergoes progressive age-dependent decline, its output is maintained until late adulthood, a finding also observed in the correlation analysis in the elderly subjects using ddPCR.

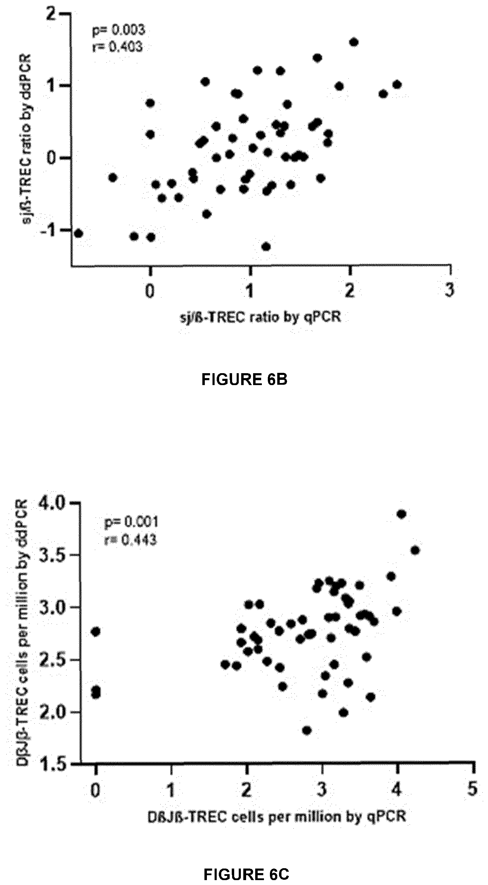

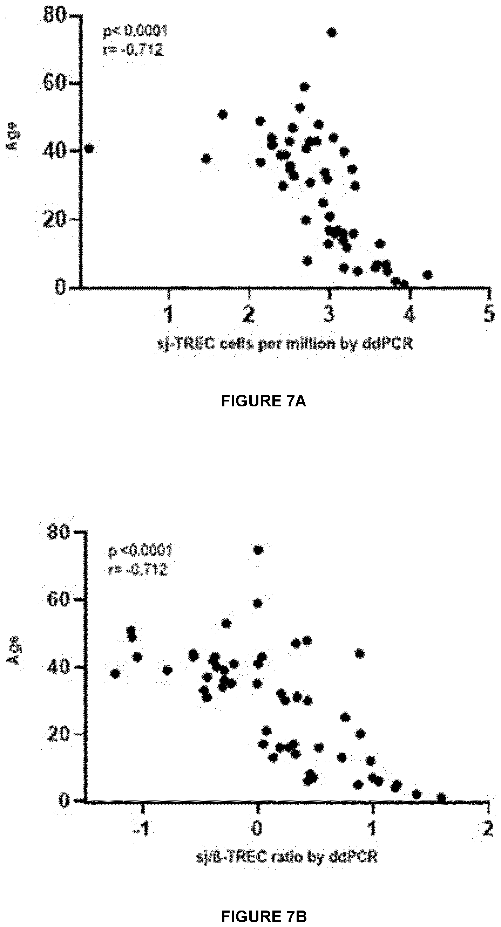

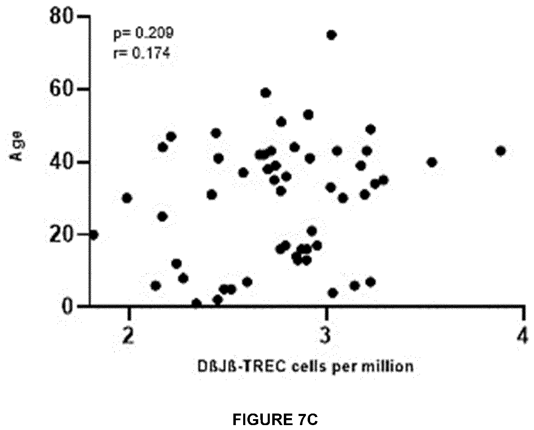

On the other hand, in the HIV-cohort analyzed in the examples included in the present disclosure (see **Example 3**), the results showed a positive correlation of sj-TREC levels, DβJβ-TREC levels and sj/DβJβ-TREC ratio using both methods. Moreover, significant correlations between thymic parameters and age were also observed (see **Example 3 and Figure 6 and 7**). Therefore, the thymic function is an important parameter and its detection by ddPCR becomes a powerful tool in early diagnosis to categorize individuals with different progression rates.

Consequently, the simplification in the detection of thymic function parameters using ddPCR includes: (i) a reduction in the number of PCR reactions and primers, which in turn save costs and time; (ii) it is not necessary to run a standard curve, which decreases the experimental errors; (iii) it is more sensitive and precise, as we demonstrated in the detection of DβJβ-TREC which are not detected by the conventional qPCR.

The development of an accurate and simplified *in vitro* and/or *ex vivo* method to measure the thymic function by ddPCR may have important implications and benefits, contributing to the design of future strategies that can maintain and/or increase the thymic function. These future strategies include the improvement of vaccine responses, the prognosis of infectious diseases, and the delay in the onset of oncological and autoimmune diseases, as well as the prevention of cardiovascular diseases by controlling systemic inflammation.

A first aspect of the present disclosure refers to a method *in vitro* and/or *ex vivo* for assessing and monitoring thymic function in an isolated biological sample from an individual, hereinafter referred to as the first method of the invention, wherein this first method comprising:

a) Measuring the expression levels of sj-TREC (SEQ ID NO: 32) and DβJβ-TREC (SEQ ID NO: 31) in the isolated sample by ddPCR;

b) Determining the sj/DβJβ-TREC ratio;

c) Repeating steps (a) and (b) using the same type of biological sample but obtained from the individual at a subsequent point in time;

d) Comparing the sj/DβJβ-TREC ratio measured in step (b) with that measured in step (c), thereby monitoring the progression or regression of thymic function in the individual.

In a second aspect, the present disclosure relates to a method *in vitro* and/or *ex vivo* for the prognosis and/or diagnosis of an immune system dysfunction in an isolated biological sample from an individual, hereinafter referred to as the second method of the invention, wherein this second method comprising:

a) Measuring the expression levels of sj-TREC (SEQ ID NO: 32) and DβJβ-TREC (SEQ ID NO: 31) in the isolated sample by ddPCR;

b) Determining the sj/DβJβ-TREC ratio;

wherein a value of said sj/DβJβ-TREC ratio different from that found in a healthy individual of the same age is an indication that the individual has an immune system dysfunction.

In a third aspect, the present disclosure relates to a method *in vitro* and/or *ex vivo* for assessing the effect of therapeutic treatment in an isolated biological sample from an individual, hereinafter referred to as the third method of the invention, wherein this third method comprising:

a) Measuring the expression levels of sj-TREC (SEQ ID NO: 32) and DβJβ-TREC (SEQ ID NO:31) in the isolated sample by ddPCR;

b) Determining the sj/DβJβ-TREC ratio;

c) Repeating steps (a) and (b) using the same type of biological sample but obtained from the individual at a subsequent point in time after to administering the therapeutic treatment;

d) Comparing the sj/DβJβ-TREC ratio measured in step (b) with that measured in step (c), thereby assessing the effect of the therapeutic treatment on the individual.

In a fourth aspect, the present disclosure also relates to the use, *in vitro* and/or *ex vivo,* of the expression level of sj-TREC (SEQ ID NO: 32), DβJβ-TREC (SEQ ID NO: 31) and sj/DβJβ-TREC ratio, obtained by ddPCR, as biomarkers for:

i. assessing and monitoring thymic function in an isolated biological sample from an individual;

ii. prognosis and/or diagnosis of an immune system dysfunction in an isolated biological sample from an individual; and/or

iii. assessing an effect of a therapeutic treatment in an isolated biological sample from an individual.

In a fifth aspect, the present disclosure also relates to a kit for assessing and monitoring thymic function, for the prognosis and/or diagnosis of an immune system dysfunction and/or for assessing the effect of a therapeutic treatment in an isolated biological sample from an individual, wherein the kit comprises reagents for amplifying the sj-TREC (SEQ ID NO: 32) and DβJβ-TREC (SEQ ID NO: 31) in said sample by ddPCR with the primers and probes set forth in **Table 3.** The probes disclosed in the present document comprise a detectable moiety and/or a quencher.

## BRIEF DESCRIPTION OF THE DRAWINGS

To complete the description and in order to provide a better understanding of the invention, a set of drawings is provided. Said drawings form an integral part of the description and illustrate aspects and embodiments disclosed herein, which should not be interpreted as restricting the scope of the invention, but just as an example of how the invention can be carried out. The drawings comprise the following figures:

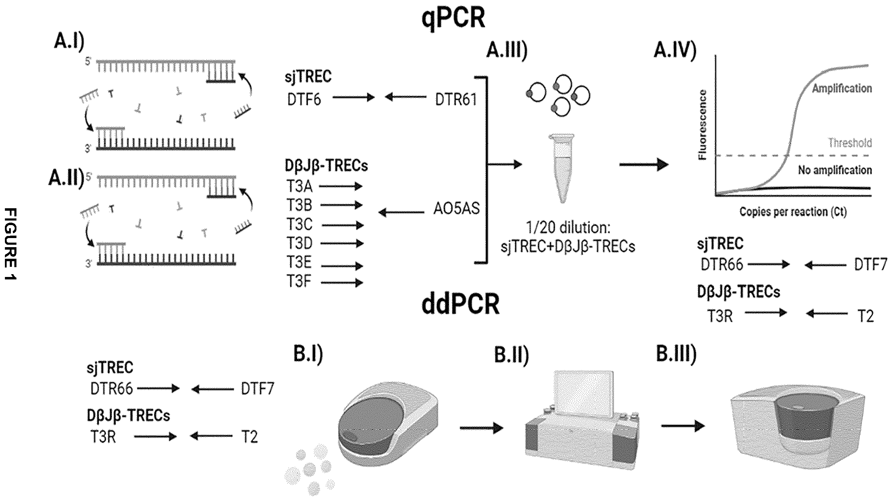

**Figure 1. Schematic representation of the quantification of sj-TREC and DβJβ-TRECs by conventional qPCR and ddPCR.** Quantification by qPCR (**A**): Amplification of sj-TREC (**A.I**) and DβJβ-TRECs (**A.II**) in two different amplification reactions. Mix and dilution of sj-TREC and DβJβ-TREC regions previously amplified (**A.III**). Amplification by qPCR (LightCycler480) (**A.IV**). Quantification by ddPCR (**B**): droplet generation by the Bio-Rad QX200 droplet generator (**B.I**), droplets amplification by PCR thermal cycler (**B.II**) and quantification by Bio-Rad droplet digital reader (**B.III**).

**Figure 2. Standard curves of DβJβ-TREC and sj-TREC levels by ddPCR.** Ten-fold dilutions of DβJβ-TREC and sj-TREC plasmids were obtained in three independent experiments. Each dot represents the copies detected in each standard curve dilution that was assayed in duplicate.

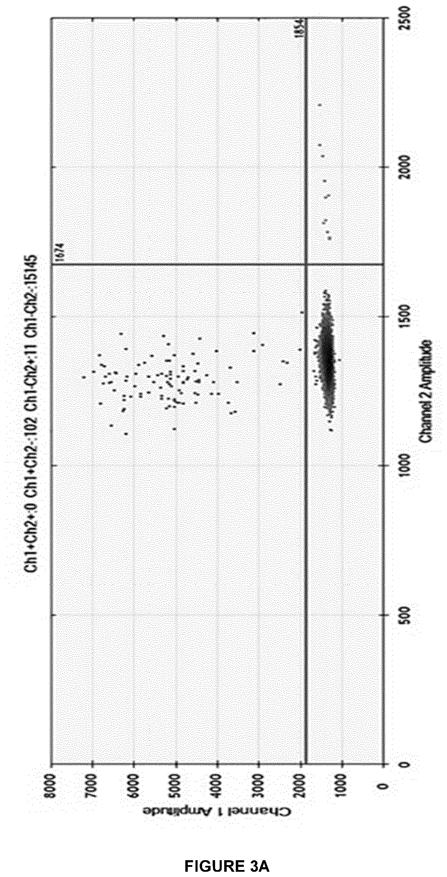

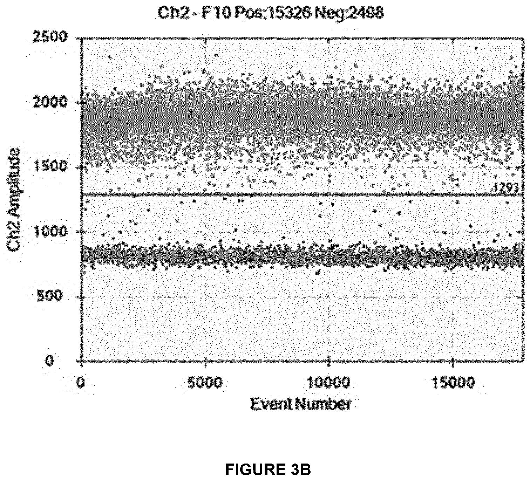

**Figure 3. Representative example of the quantification of DβJβ-TRECs and sj-TREC (A) and RPP30 (B) by Bio-Rad QuantaSoft software v.1.7.4. (A)** Channel 1 measured DβJβ-TREC regions (upper left quadrant), channel 2 measured sj-TREC regions (lower right quadrant). **(B)** Channel 2 measured RPP30 copies (upper quadrant).

**Figure 4. Correlations between thymic function measurements, using qPCR and ddPCR in elderly healthy donors.** Dot-plot graphs of sj-TREC (**A**), sj/DβJβ-TREC ratio (**B**) and DβJβ-TREC (**C**) cells per million. Results are represented in logarithmic scale. Each dot represents an individual. Spearman test was used for non-parametric correlations.

**Figure 5. Correlations between thymic function measurements and age in elderly healthy donor.** Dot plot graphs of sj/DβJβ-TREC ratio by qPCR (**A**) and sj/DβJβ-TREC ratio by ddPCR (**B**). Each dot represents an individual. Spearman test was used for non-parametric correlations.

**Figure 6. Correlations between thymic function measurements using qPCR and ddPCR in people living with HIV.** Dot plot graphs of sj-TREC (**A**), sj/DβJβ-TREC ratio (**B**) and DβJβ-TREC (**C**) cells per million. Results are expressed in logarithmic scale. Each dot represents an individual. Spearman test was used for non-parametric correlations.

**Figure 7. Correlations between thymic function measurements and age in people living with HIV by ddPCR.** Dot plots of sj-TREC (**A**), sj/DβJβ-TREC ratio (**B**) and DβJβ-TREC (**C**) cells per million. Results are expressed in logarithmic scale. Each dot represents an individual. Spearman test was used for non-parametric correlations.

## DETAILED DESCRIPTION OF THE INVENTION.

The following definitions and methods are provided to better define the present invention and to guide those of ordinary skill in the art in the practice of the present invention. Unless otherwise noted, terms are to be understood according to conventional usage by those of ordinary skill in the relevant art. Each aspect so defined may be combined with any other aspect or aspects unless clearly indicated to the contrary. In particular, any feature indicated as being preferred or advantageous may be combined with any other feature or features indicated as being preferred or advantageous.

In a first aspect, the present disclosure relates to a method *in vitro* and/or *ex vivo* for assessing and monitoring thymic function in an isolated biological sample from an individual, hereinafter referred to as the first method of the invention, wherein this first method comprises:

a) Measuring the expression levels of sj-TREC (SEQ ID NO: 32) and DβJβ-TREC (SEQ ID NO: 31) in the isolated sample by ddPCR;

b) Determining the sj/DβJβ -TREC ratio;

c) Repeating steps (a) and (b) using the same type of biological sample but obtained from the individual at a subsequent point in time;

d) Comparing the sj/DβJβ-TREC ratio measured in step (b) with that measured in step (c), thereby monitoring the progression or regression of thymic function in the individual.

In a second aspect, the present disclosure relates to a method *in vitro* and/or *ex vivo* for the prognosis and/or diagnosis of an immune system dysfunction in an isolated biological sample from an individual, hereinafter referred to as the second method of the invention, wherein this second method comprises:

a) Measuring the expression levels of sj-TREC (SEQ ID NO: 32) and DβJβ-TREC (SEQ ID NO: 31) in the isolated sample by ddPCR;

b) Determining the sj/DβJβ-TREC ratio;

wherein a value of said sj/DβJβ-TREC ratio different from that found in a healthy individual of the same age is an indication that the individual has an immune system dysfunction.

In a third aspect, the present disclosure relates to a method *in vitro* and/or *ex vivo* for assessing the effect of a therapeutic treatment in an isolated biological sample from an individual, hereinafter referred to as the third method of the invention, wherein this third method comprises:

a) Measuring the expression levels of sj-TREC (SEQ ID NO: 32) and DβJβ-TREC (SEQ ID NO: 31) in the isolated sample by ddPCR;

b) Determining the sj/DβJβ-TREC ratio;

c) Repeating steps (a) and (b) using the same type of biological sample but obtained from the individual at a subsequent point in time after to administering the therapeutic treatment;

d) Comparing the sj/DβJβ-TREC ratio measured in step (b) with that measured in step (c), thereby assessing the effect of the therapeutic treatment on the individual.

As used herein, the terminology "thymic function" is meant to refer to the level of thymic activity, thus the quantity of output of recent T cells from the thymus to peripheral blood and/or secondary lymphoid organs and/or any other tissue containing lymphocytes.

As it is known in the art, two different types of TRECs are usually quantified to determine the thymic function, and therefore, to carry out the prognosis and/or diagnosis of immune system dysfunction in an individual. The six DβJβ-TRECs which are a product of the β chain TCR rearrangement at the most immature thymocyte subset and are included in cluster 1, have the sequence deposited in the GenBank database (NCBI) with accession number No. L36092.2 and in SEQ ID NO: 31. The sj-TREC is a product of the α chain TCR rearrangement and has the sequence deposited in the GenBank database (NCBI) with accession number No. AE000521.1 and in SEQ ID NO: 32.

As used herein, the term "level of expression" or "expression levels" refers to the measurable expression level of a given nucleic acid or polypeptide. The level of expression of a given nucleic acid or polypeptide is determined by methods well-known in the art. In a preferred embodiment of the present invention, the expression level of DβJβ-TRECs and sj-TREC are measured by ddPCR. The term "differentially expressed" or "differential expression" refers to an increase or decrease in the measurable expression level of a given nucleic acid or polypeptide. "Differentially expressed" or "differential expression" means a 1-fold, or more, up to and including 2-fold, 3-fold, 4-fold, 5-fold, 6-fold, 7-fold, 8-fold, 9-fold, 10-fold, 20-fold, 50-fold or more difference in the level of expression of a given nucleic acid or polypeptide in two samples used for comparison. A given nucleic acid or polypeptide is also said to be "differentially expressed" in two samples if one of the two samples contains no detectable expression of a given nucleic acid or polypeptide.

As used herein the terminology "biological sample" refers to any biological material isolated from a subject, including any biological fluid or tissue that contains immune cells. The most suitable biological sample for use in the methods disclosed herein includes blood, preferably, peripheral blood, and more preferably peripheral blood mononuclear cells (PBMCs). Other useful biological samples include, without limitation, whole blood, saliva, urine, synovial fluid, bone marrow, cerebrospinal fluid, and other cellular exudates from a patient. Such samples may further be diluted with saline, buffer or a physiologically acceptable diluent.

As used herein the terminology "same type of biological sample" as used in descriptions of methods of present disclosure means that when the biological sample obtained at the first point in time is blood, for instance, the sample obtained at the second point in time is also blood. This enables the measures obtained at each two points in time to be comparable.

As used herein the terminology "individual", "subject" or "patient" refers to a mammalian animal, including a human, a veterinary or farm animal, a domestic animal or pet, and animals normally used for clinical research. In one embodiment, the subject of these methods and uses is a human, that is healthy or diseased.

A used herein the terminology "immune system dysfunction" refers to any clinical manifestation of subnormal or supra-normal numbers, activation levels, production rate and/or death rate of lymphocytes or other immune cells. Without being so limited, this terminology includes pathologies such as acute and chronic viral infections (HIV and AIDS, HCV, EBV, etc.), autoimmune diseases (multiple sclerosis, Crohn's disease), autoimmune endocrine disorders (autoimmune polyglandular syndromes, Graves' disease, immune-mediated diabetes, etc.) rheumatoid arthritis, systemic lupus erythematosus, etc.), cancers (all forms, before and after chemotherapy). A transient immune system dysfunction is also found in patients undergoing the immune reconstitution through bone marrow transplantation (BMT) and allogeneic haematopoietic stem cell transplantation (AHSCT) following myeloablative regimen treatment for hematological malignancies and possibly in other of the diseases mentioned above.

As used herein, the terminology "treatment" refers to inhibiting, preventing, or arresting the development of a pathology (disease, disorder, or condition) and/or causing the reduction, remission, or regression of a pathology. Those with skill in the art will understand that various methodologies and assays can be used to assess the development of a pathology, and similarly, various methodologies and assays may be used to assess the reduction, remission, or regression of a pathology.

As used herein the phrase "treatment regimen" refers to a treatment plan that specifies the type of treatment, dosage, schedule, and/or duration of a treatment provided to a subject in need thereof. The selected treatment regimen can be an aggressive one that is expected to result in the best clinical outcome (e.g., complete cure of the pathology), yet may be associated with some discomfort to the subject or adverse side effects (e.g., a damage to healthy cells or tissue); or a more moderate one which may relief symptoms of the pathology yet may result in incomplete cure of the pathology. The type of treatment, dosage, schedule, and duration of treatment can vary, depending on the severity of pathology and the predicted responsiveness of the subject to the treatment, and those of skills in the art are capable of adjusting the type of treatment with the dosage, schedule, and duration of treatment.

As used herein, the terminology "diagnosis" refers to the capacity to differentiate between individuals suffering from a disease or disorder, such as an immune system dysfunction, and those who are not. It also refers, without limitation, to the capacity to differentiate between samples originating from individuals or patients and samples originating from healthy individuals. This differentiation, as is understood by one skilled in the art, does not seek to be correct for all the analyzed samples. However, it does require a statistically significant amount of the analyzed samples to be correctly classified. The statistically significant amount can be established by one skilled in the art by using different statistical tools, for example, but without limitation, by means of determining confidence intervals, determining the p-value, Student's t-test, or Fisher's discriminant functions. The confidence intervals are preferably at least 90%, at least 95%, at least 97%, at least 98%, or at least 99%. The p-value is preferably less than 0.1, 0.05, 0.01, 0.005, or 0.0001. The present invention preferably allows correctly detecting the disease in a differential manner in at least 60%, in at least 70%, in at least 80%, or in at least 90% of the subjects of a specific group or analyzed population.

As used herein, the terminology "prognosis" is understood as the expected evolution of a disease and refers to the assessment of the probability according to which a subject suffers from a disease as well as the assessment of its onset, developmental status, evolution, or its regression, and/or the prognosis of the course of the disease in the future. As those skilled in the art will understand, such assessment, although preferred, may not be correct for 100% of the subjects. The term, however, requires that a statistically significant part of the subjects can be identified as having the disease or having a predisposition to it. If a part is statistically significant, it can be determined by the person skilled in the art using several well-known statistical evaluation tools, for example, determination of confidence intervals, determination of p-values, Student's t-test, Mann-Whitney test, etc. Preferred confidence intervals are at least 50%, at least 60%, at least 70%, at least 80%, at least 90%, at least 95%. P values are preferably 0.2, 0.1, 0.05.

As used herein, the terminology "prediction of the response" means the determination of the probability that the patient responds favorably or unfavorably to a particular therapy or treatment, including surgical treatment. Especially, the term "prediction", as used herein, refers to an individual evaluation of any parameter that may be useful in determining the evolution of a patient. As those skilled in the art will understand, the prediction of the clinical response to treatment, although it is preferred, does not need to be correct for 100% of the subjects to be diagnosed or evaluated. The term, however, requires that a statistically significant part of the subjects can be identified as having an increased probability of having a positive response. The person skilled in the art can easily determine if a subject is statistically significant using several well-known statistical evaluation tools, for example, determination of confidence intervals, determination of p values, Student's t-test, Mann Whitney test, etc. Preferred confidence intervals are at least 50%>, at least 60%>, at least 70%>, at least 80%>, at least 90%), at least 95%>. P values are preferably 0.2, 0, 1 or 0.05. The prediction of the clinical response can be made using any assessment criteria used in immunology and known to the person skilled in the art. In the context of the present invention the term "beneficial response" or "positive response" means an improvement in any measure of patient status including those measures ordinarily used in the art such as overall survival, long-term survival, recurrence-free survival, and distant recurrence-free survival. Recurrence-free survival (RFS) refers to the time (in years) from surgery or other treatment to the first local, regional, or distant recurrence. Distant recurrence-free survival (DFRS) refers to the time (in years) from surgery to the first distant recurrence. Recurrence refers to RFS and/or DFRS as evidenced by its particular usage. The calculation of these measures in practice may vary from study to study depending on the definition of events to be either censored or not considered. The term "long-term" survival is used herein to refer to survival for at least 3 years, more preferably for at least 8 years, and most preferably for at least 10 years following surgery or other treatment.

In a preferred embodiment, the methods of the present disclosure are characterized in that the measure of the expression level of sj-TREC and the DβJβ-TREC is performed by using specific primers and probes.

As used herein, the term "oligonucleotide" refers to linear oligomers of natural or modified monomers or linkages, including deoxyribonucleosides, ribonucleosides, and the like, capable of specifically binding to a target polynucleotide by way of a regular pattern of monomer-to-monomer interactions, such as Watson-Crick type base pairing. For purposes herein, the term oligonucleotide includes both oligonucleotide probes and oligonucleotide primers. As used herein, the term "primer" refers to an oligonucleotide that hybridizes to the template strand of a nucleic acid and initiates the synthesis of a nucleic acid strand complementary to the template strand when placed under conditions in which synthesis of a primer extension product is induced. Such conditions include the presence of nucleotides and a polymerization-inducing agent such as a DNA or RNA polymerase and at suitable temperature, pH, metal concentration, and salt concentration. For instance, a "primer" is complementary to a template, and complexes by hydrogen bonding or hybridization with the template to give a primer/template complex for initiation of synthesis by a polymerase, which is extended by the addition of covalently bonded bases linked at its 3' end complementary to the template in the process of DNA or RNA synthesis. As employed herein, an oligonucleotide primer can be naturally occurring, as in a purified restriction digest, or can be produced synthetically. The primer is preferably single-stranded for maximum efficiency in amplification. In some cases, a primer may alternatively be double-stranded. If double-stranded, the primer can first be treated to separate its strands before being used to prepare extension products. This denaturation step is typically effected by heat, but may alternatively be carried out using alkali, followed by neutralization.

As used herein, "primer pair" refers to two primers, a forward primer and a reverse primer, that are capable of participating in PCR amplification of a segment of nucleic acid in the presence of a nucleic acid polymerase to produce a PCR amplicon. It is understood that the orientation of the primers is the direction in which the elongation of the primer in DNA synthesis occurs. Since DNA synthesis is 5' to 3', the 3' ends of a PCR primer set (e.g. forward primer and reverse primer) point towards each other, when they are annealed to their template strand, and the primers anneal on opposite strands of the PCR template. For instance, the forward primer may anneal to (i.e. is complementary to) the minus template minus (-) strand and the reverse primer anneals to (i.e. is complementary to) the template (+) strand.

As used herein, a "detectable moiety" or "detectable label" refers to a molecule capable of detection, including, but not limited to, radioactive isotopes, fluorophores, chemiluminescent, chromophores, enzymes, enzyme substrates, enzyme co-factors, enzyme inhibitors, semiconductor nanoparticles, dyes, metal ions, metal sols, ligands (e.g., biotin, streptavidin or haptens) and the like. The term "fluorescent moiety" refers to a substance or a portion thereof that is capable of exhibiting fluorescence in the detectable range. As used herein, "fluorophore" refers to a fluorescent reporter molecule which, upon excitation with a laser, tungsten, mercury or xenon lamp, or a light emitting diode, releases energy in the form of light with a defined spectrum. Through the process of fluorescence resonance energy transfer (FRET), the light emitted from the fluorophore can excite a second molecule whose excitation spectrum overlaps the emission spectrum of the fluorophore. The transfer of emission energy of the fluorophore to another molecule quenches the emission of the fluorophore. The second molecule is known as a quencher molecule. The term "fluorophore" is used interchangeably herein with the term "fluorescent reporter".

As used herein "quencher" or "quencher molecule" refers to a molecule that, when linked to a fluorescent probe comprising a fluorophore, is capable of accepting the energy emitted by a fluorophore, thereby quenching the emission of the fluorophore. A quencher can be fluorescent, which releases the accepted energy as light, or non-fluorescent, which releases the accepted energy as heat and can be attached at any location along the length of the probe.

As used herein, "probe" refers to an oligonucleotide that is capable of forming a duplex structure with a sequence in a target nucleic acid, due to the complementarity of at least one sequence of the probe with a sequence in the target region, or region to be detected. The term "probe" includes an oligonucleotide as described above, with or without a fluorophore and a quencher molecule attached. The term "fluorescent probe" refers to a probe comprising a fluorophore and a quencher molecule.

In a preferred embodiment of the present invention the primers and probes used for measuring the expression level of sj-TREC and the DβJβ-TREC are set for below:

sj-TREC primers and probe:

- DTF7 primer having the SEQ ID NO 10: AGGCTCTGTCTAGTGTGATAAC.

- DTR66 primer having the SEQ ID NO: 11: TGACATGGAGGGCTGAAC.

- SD1 probe having the SEQ ID NO: 26: CACCCCTCTGTTCCCCACA.

DβJβ-TRECs primers and probe:

- T3A primer having the SEQ ID NO:20: CTTTCGATGGACCCTCACAG.

- T3B primer having the SEQ ID NO: 21: GACAAGGCACCAGACTCACAG.

- T3C primer having the SEQ ID NO: 22: AAGCTCTGGAAGGGAACACAG.

- T3D primer having the SEQ ID NO: 23: CCGTTTCTCTCCCTCACACAG.

- T3E primer having the SEQ ID NO: 24: GGGCAGAAACTGAGAACACAG.

- T3F primer having the SEQ ID NO: 25: CTTGCGCCTTATGCTGCACAG.

- T2 primer having the SEQ ID NO: 13: CCCAGGAGGAAAGAAGAGGAC.

- PB1 probe having the SEQ ID NO: 27: TGGGAGTTGGGACCGCCAGAGAGG.

The provided probes mentioned above may be labeled with different detectable means. This detectable means refers to compounds, biomolecules or biomimetics that can be conjugated, connected, or attached to probes to provide quantitative indices such as density, concentration, quantity, etc. Examples of detectable means include fluorescent markers, fluorophores, luminescents, bioluminescents, and radio isotopes, but are not limited thereto. If used together, two or more fluorescent markers may be different in color. Details and selection of the fluorescent markers are known to those skilled in the art (See e.g., Bao et al., Annual Review of Biomedical Engineering (11): 25-47, 2009).

In some embodiments, the oligonucleotide probe is labeled at the 5' end with a fluorescent marker selected from the group consisting of FAM, VIC, TET, JOE, HEX, CY3, CY5, ROX, RED610, TEXAS RED, RED670, NED, FITC, TAMRA, ABY, PET, JUN, LIZ, LC640, or any combinations thereof. In some embodiments, the oligonucleotide probe is labeled at the 3' end with a fluorescent marker selected from the group consisting of FAM, VIC, TET, JOE, HEX, CY3, CY5, ROX, RED610, TEXAS RED, RED670, NED, FITC, TAMRA, ABY, PET, JUN, LIZ, LC640, PH, FL, Red705, or any combinations thereof. In a preferred embodiment, the probe is labeled at the 5'end with a fluorescent marker selected from the group consisting of FAM, VIC, TET, JOE, HEX, CY3, CY5, ROX, RED610, TEXAS RED, RED670, NED, FITC, TAMRA, ABY, PET, JUN, LIZ, LC640, PH, FL, Red705, or any combinations thereof.

In some embodiments, the oligonucleotide probe is labeled at the 3' end with a fluorescence quencher. In some embodiments, the quencher is part of a fluorescent probe comprising a fluorophore and is capable of accepting the energy emitted by the fluorophore, thereby quenching the emission of the fluorophore. A quencher can be fluorescent, which releases the accepted energy as light, or non-fluorescent, which releases the accepted energy as heat, and can be attached at any location along the length of the probe. In some embodiments, the oligonucleotide probe is labeled at the 5' end with a fluorescence quencher. In some embodiments, the quencher is selected from the group consisting of 6-TAMRA, BHQ-1,2,3 and MGBNFQ. In some embodiments, the oligonucleotide probe is labeled at the 3' end with a fluorescence quencher. In some embodiments, the quencher is selected from the group consisting of 6-TAMRA, BHQ-1,2,3 and MGBNFQ. In a preferred embodiment, the oligonucleotide probe is labeled at the 3' end with a fluorescence quencher. In some embodiments, the quencher is selected from the group consisting of 6-TAMRA, BHQ-1,2,3 and MGBNFQ.

Oligonucleotide probes and primers provided herein can be synthesized by a number of methods. See, e.g., Ozaki et al., Nucleic Acids Research 20: 5205-5214 (1992); Agrawal et al., Nucleic Acids Research 18: 5419-5423 (1990). Among the methods are methods involving solid phase synthesis via phosphoramidite chemistry, such as disclosed in U.S. Patent Nos. 4,458,066 and 4,415,732, incorporated herein by reference; Beaucage et al, Tetrahedron (1992) 48:2223-2311; and Applied Biosystems User Bulletin No. 13 (1 April 1987). Other chemical synthesis methods include, for example, the phosphotriester method described by Narang et al, Meth. Enzymol. (1979) 68:90 and the phosphodiester method disclosed by Brown et al, Meth. Enzymol. (1979) 68: 109. Poly(A) or poly(C), or other non-complementary nucleotide extensions may be incorporated into oligonucleotides using these same methods. Hexaethylene oxide extensions may be coupled to the oligonucleotides by methods known in the art. Cload et al, J. Am. Chem. Soc. (1991) 113:6324-6326; U.S. Patent No. 4,914,210 to Levenson et al; Durand et al, Nucleic Acids Res. (1990) 18:6353-6359; and Horn et al, Tet. Lett. (1986) 27:4705-4708. In an example, oligonucleotide probes can be synthesized on an automated DNA synthesizer such as the ABI 3900 DNA Synthesizer (Applied Biosystems, Foster City, CA). Alternative chemistries, e.g. resulting in non-natural backbone groups, such as phosphorothioate, phosphoramidate, and the like, may also be employed provided that the hybridization efficiencies of the resulting oligonucleotides are not adversely affected.

In a more preferred embodiment, the fluorescent probe SD1 (SEQ ID NO: 26) is labeled at the 5' end with the fluorescent marker HEX, and at the 3'end with the fluorescence quencher BHQ-1.

In a more preferred embodiment, the fluorescent probe PB1 (SEQ ID NO: 27) is labeled at the 5' end with the fluorescent marker 6-FAM, and at the 3'end with the fluorescence quencher BHQ-1.

In addition, any housekeeping gene known in the art can be used in the method of the present invention, and the design of the primers and probes for the detection of the expression levels of these housekeeping genes is known to those of ordinary skill in the art. Among the housekeeping genes that may be useful for the methods of the present invention, any of the following may be selected the Rribonuclease P/MRP subunit p30 (RPP30), Actin beta (ACTB), Glyceraldehyde-3-phosphate dehydrogenase (GAPDH), Ribosomal Protein L4 (RPL4) and Ribosomal Protein S18 (RPS18), among others. In a preferred embodiment, the RPP30 was used as a housekeeping gene and the primers for measuring the expression level of RPP30 are set forth as SEQ ID Nos: 28 (GATTTGGACCTGCGAGCG) and 29 (GCGGCTGTCTCCACAAGT), and the probe is set forth as SEQ ID NO: 30 (CTGACCTGAAGGCTCT). In a preferred embodiment, the fluorescent probe RPP30 (SEQ ID NO: 30) is labeled at the 5' end with the fluorescent marker VIC, and at the 3' end with the fluorescence quencher BHQ-1.

In another preferred embodiment, the methods of the present invention are characterized in that the assessment and monitoring are for evaluating the efficacy of a therapy; the side effects of a therapy, and/or the effect on the thymic function of a disorder or disease.

In another preferred embodiment, the therapy's efficacy is assessed and monitored from a therapy selected from the group consisting of vaccination, antiviral therapy, T-cell reconstitutions, bone marrow transplantation and hematopoietic stem cell transplantation.

In another preferred embodiment, the therapy's side effects are assessed and monitored from a therapy selected from the group consisting of radiotherapy, chemotherapy and drug therapy.

In another preferred embodiment of the methods of the present disclosure, they are characterized in that the individual has an immune system dysfunction, so it is an immunocompromised individual. In a preferably embodiment, the immune system dysfunction is selected from the list consisting of: acute and chronic viral infections, (HIV and AIDS, HCV, EBV, etc.), autoimmune diseases (multiple sclerosis, Crohn's disease, etc.), autoimmune endocrine disorders (autoimmune polyglandular syndromes, Graves' disease, immune mediated diabetes, etc.) rheumatoid arthritis, systemic lupus erythematosus, etc., cancers (all forms, before and after chemotherapy), bone marrow transplantation (BMT), organ transplantation and allogeneic haematopoietic stem cell transplantation (AHSCT).

The definitions mentioned herein, apply *mutatis mutandis* to all the aspect disclosed in the present specification.

In a fourth aspect, the present disclosure also relates to the use, *in vitro* and/or *ex vivo,* of the expression level of sj-TREC (SEQ ID NO: 32), DβJβ-TREC (SEQ ID NO: 31) and sj/DβJβ-TREC ratio, obtained by ddPCR, as biomarkers for:

    i. assessing and monitoring thymic function in an isolated biological sample from an individual;

    ii. prognosis and/or diagnosis of an immune system dysfunction in an isolated biological sample from an individual; and/or

    iii. assessing the effect of a therapeutic treatment in an isolated biological sample from an individual.

In a preferred embodiment of the use of the present disclosure, it is characterized by the primers and probes used for measuring said expression levels are set below:

sj-TREC primers and probe:

-    DTF7 primer having the SEQ ID NO 10: AGGCTCTGTCTAGTGTGATAAC.

-    DTR66 primer having the SEQ ID NO: 11: TGACATGGAGGGCTGAAC.

- SD1 probe having the SEQ ID NO: 26: CACCCCTCTGTTCCCCACA.

DβJβ-TRECs primers and probe:

- T3A primer having the SEQ ID NO:20: CTTTCGATGGACCCTCACAG.

- T3B primer having the SEQ ID NO: 21: GACAAGGCACCAGACTCACAG.

- T3C primer having the SEQ ID NO: 22: AAGCTCTGGAAGGGAACACAG.

- T3D primer having the SEQ ID NO: 23: CCGTTTCTCTCCCTCACACAG.

- T3E primer having the SEQ ID NO: 24: GGGCAGAAACTGAGAACACAG.

- T3F primer having the SEQ ID NO: 25: CTTGCGCCTTATGCTGCACAG.

- T2 primer having the SEQ ID NO: 13: CCCAGGAGGAAAGAAGAGGAC.

- PB1 probe having the SEQ ID NO: 27: TGGGAGTTGGGACCGCCAGAGAGG.

In a more preferred embodiment, the fluorescent probe SD1 (SEQ ID NO: 26) is labeled at the 5' end with the fluorescent marker HEX, and at the 3'end with the fluorescence quencher BHQ-1.

In a more preferred embodiment, the fluorescent probe PB1 (SEQ ID NO: 27) is labeled at the 5' end with the fluorescent marker 6-FAM, and at the 3'end with the fluorescence quencher BHQ-1.

In addition, as previously mentioned, any housekeeping gene known in the art can be used in the method of the present invention, and the design of the primers and probes for the detection of the expression levels of these housekeeping genes is known to those of ordinary skill in the art. Among the housekeeping genes that may be useful for the methods of the present invention, any of the following may be selected the Rribonuclease P/MRP subunit p30 (RPP30), Actin beta (ACTB), Glyceraldehyde-3-phosphate dehydrogenase (GAPDH), Ribosomal Protein L4 (RPL4) and Ribosomal Protein S18 (RPS18), among others. In a preferred embodiment, the RPP30 gene was used as a housekeeping gene and the primers for measuring the expression level of RPP30 are set forth as SEQ ID Nos: 28 (GATTTGGACCTGC GAGCG) and, 29 (GCGGCTGTCTCCACAAGT), and the probe is set forth as SEQ ID NO: 30 (VIC-CTGACCTGAAG GCTCT-BHQ1). In a preferred embodiment, the fluorescent probe RPP30 (SEQ ID NO: 30) is labeled at the 5' end with the fluorescent marker VIC, and at the 3'end with the fluorescence quencher BHQ-1.

In a further aspect, the present disclosure also relates to a kit for assessing and monitoring thymic function, for the prognosis and/or diagnosis of an immune system dysfunction and/or for assessing an effect of a therapeutic treatment in an isolated biological sample from an individual, wherein the kit comprises reagents for amplifying the sj-TREC (SEQ ID NO: 32) and DβJβ-TREC (SEQ ID NO: 31) in said sample by ddPCR by the primers and probes mentioned previously, such as SEQ ID NOs: 10, 11 as primers and SEQ ID NO: 26 as a probe for measuring the expression level of sj-TREC and SEQ ID Nos: 13, 20 to 25 as primers, and SEQ ID NO: 27 as a probe for measuring the expression level of DβJβ-TRECs.

## EXAMPLES

Following are examples of the invention by means of assays carried out by the inventors, which evidence the effectiveness of the product of the invention. The following examples serve to illustrate the invention and must not be considered to limit the scope thereof.

MATERIALS AND METHODS

*Study participants*

Peripheral blood samples were obtained from 26 elderly healthy donors (inclusion criteria: $\geq$ 65 years old and self-sufficient health status; exclusion criteria: diagnosis of dementia, active infections, hospital admission, antitumor therapy, or any treatment that could influence their immune status during the 6 months before the extraction) and 54 people living with HIV (PLHIV) classified as typical progressors, long-term non-progressors (LTNPs), and vertically HIV-infected. These blood samples were obtained from Gregorio Marañon Hospital Biobank (Madrid, Spain) which is integrated into the Spanish AIDS Research Network (RIS) (Garcia-Merino I, et al. Retrovirology. 2009;6:1-5). Progressor cohort (n=15) was selected from the cohort of adults with HIV infection of RIS (CoRIS), newly diagnosed subjects naive for antiretroviral treatment. LTNPs cohort (n=18) was selected following the immunological criteria: CD4+ T-cell counts > 500 cells/ μL, more than 10 years after HIV diagnosis without antiretroviral treatment and plasma HIV loads between 50 and 5000 HIV-RNA copies/ml. The pediatric cohort (CoRISpe) (n=21) was based on individuals vertically HIV-infected under 18 years of age, HIV-RNA in peripheral blood detected, with at least one pediatric visit since January 1995 and at least one follow-up time point. All individuals, or their legal caretakers, were informed and gave their approval to participate. The study was reviewed and approved by the scientific and ethic committees of Virgen del Rocio University Hospital and RIS Spanish HIV-HGM Biobank.

*PBMCs isolation and DNA extraction*

Peripheral blood mononuclear cells (PBMCs) were isolated from approximately 64 millilitres of whole blood samples by using BD Vacutainer® CPT™ Mononuclear Cell Preparation Tubes (BD Biosciences), with sodium heparin as anticoagulant, and then used for DNA extraction. The extraction of the genomic DNA, using a blood DNA minikit (Omega Bio-Tek), and its quantification, using the Qubit assay (Thermo Fischer Scientific), were performed according to the manufacturer's instructions.

*sj/DβJβ-TREC ratio measurement by qPCR*

The six DβJβ-TRECs (Dβ1-Jβ1 rearrangements) (SEQ ID NO: 31) and sj-TREC (δrec-ψJα rearrangement) (SEQ ID NO: 32) were amplified independently using a DNA concentration of at least 6 ng as follows: 10 minutes (min) at 95°C, 20 amplification cycles at 95°C for 20 seconds (sec), 57°C for 45 see and 72°C for 30 sec, and a 5 min hold at 72°C. Measurements were performed in triplicates using KAPA HiFi DNA polymerase (Roche). The sj-TREC primers (sense: DTF6; antisense: DTR61) were used at a concentration of 200 nM (**Figure 1A.I**); DβJβ-TRECs sense (T3A, T3B, T3C, T3D, T3E and T3F) and DβJβ-TRECs antisense (AO5AS) primers were used at a concentration of 80 nM and 280 nM (**Figure 1A.II**), respectively. The sequences of the primers are shown in **Table 1.**

**Table 1. Specific primers for the amplification of sj-TRECs and DβJβ-TRECs by conventional PCR.** The sequence direction is from the 5' to the 3' end.

| sj-TREC primers | Sequence (5'→3') | SEQ ID NO: |
|---|---|---|
| DTF6 | AGAAGGCTCTGTCTAGTGTG | 1 |
| DTR61 | TCTGACATTTGCTCCGTG | 2 |
| **DβJβ-TRECs primers** | **Sequence (5'→3')** | **SEQ ID NO:** |
| T3A | CGGCCGTAGCGACGTACCCTTTCGATGGACCCTCACAG | 3 |
| T3B | CGGCCGTAGCGACGTACCGACAAGGCACCAGACTCACAG | 4 |
| T3C | CGGCCGTAGCGACGTACCAAGCTCTGGAAGGGAACACAG | 5 |
| T3D | CGGCCGTAGCGACGTACCCCGTTTCTCTCCCTCACACAG | 6 |
| T3E | CGGCCGTAGCGACGTACCGGGCAGAAACTGAGAACACAG | 7 |
| T3F | CGGCCGTAGCGACGTACCCTTGCGCCTTATGCTGCACAG | 8 |
| ASO5AS | TGAACCAAATTGCATTAAGACC | 9 |

Then, a second round of amplification was performed for the combination of DβJβ and sj-TRECs by using the LightCycler® 2.0 Real-Time PCR System (Roche). A 1/20 dilution was prepared by mixing 10 μl of the previously amplified DβJβ-TRECs and sj-TRECs with 180 μL of PCR grade water (**Figure 1A.III**). Three microliters of this mix were amplified in

a 20 μL final volume using LightCycler® FastStart DNA MasterPLUS HybProbe (Roche) according to the manufacturers' instructions.

The sj-TREC primers (DTR66 and DTF7) were used at a concentration of 100 nM while DβJβ-TRECs primers (T3R and T2) were used at 200 nM (**Figure 1A.IV**), in combination with the primers sj-TRECs and DβJβ-TRECs amplification disclosed in **Table 1.** The sequence of the primers and probes is listed in **Table 2.** In this case, the PCR conditions were: 10 min at 95°C, 45 amplification cycles of 95°C for 10 sec, 57°C for 20 sec, and 72°C for 15 sec. β-globin was used as a reference gene with the primers GH20 and PC04. A standard curve was designed for the quantification of sj-TREC and DβJβ-TREC regions.

**Table 2. Specific primers for the quantification of sj-TRECs and DβJβ-TRECs by qPCR.** The sequence direction is from the 5' to the 3' end.

| sj-TREC primers | Sequence (5'→3') | SEQ ID NO: |
|---|---|---|
| DTF7 | AGGCTCTGTCTAGTGTGATAAC | 10 |
| DTR66 | TGACATGGAGGGCTGAAC | 11 |
| **DβJβ-TRECs primers** | **Sequence (5'→3')** | **SEQ ID** NO: |
| T3R | CGGCCGTAGCGACGTACC | 12 |
| T2 | CCCAGGAGGAAAGAAGAGGAC | 13 |
| **β-globin primers** | **Sequence (5'→3')** | **SEQ ID NO:** |
| GH20 | GAAGAGCCAAGGACAGGTAC | 14 |
| PC04 | CAACTTCATCCACGTTCACC | 15 |
| **sj-TREC Probes** | **Sequence (5'→3')** | **SEQ ID NO:** |
| TCRFL | AGGGATGTGGCATCACCTTTGTTGACA | 16 |
| TCRLC | GGCACCCCTCTGTTCCCCACAGGA | 17 |
| **DβJβ-TRECs Probes** | **Sequence (5'→3')** | **SEQ ID NO:** |
| P1 | CTGGGAGTTGGGACCGCCAGAGAGGT | 18 |
| P2 | TTTGTAAAGGTTTCCCGTAGAGTTGAATCATTGTG | 19 |

The probes have a detectable moiety, thus the TCRFL probe of SEQ ID NO: 16 is labeled at the 3' end with the fluorescent probe FL. The TCRLC probe of SEQ ID NO: 17 is labeled at the 5' end with the fluorophore LC640 and at the 3'end with the fluorescence quencher. The P1 probe of SEQ ID NO: 18 is labeled at the 3'end with the fluorophore FL and the P2 probe of SEQ ID NO: 19 is labeled at the 5' end with the fluorophore Red705 and at the 3' end with the fluorescent quencher PH.

*Standard curves for sj and DβJβ-TRECs quantification by qPCR*

The specific primers used for the amplification of sj-TREC (δrec-ψJα rearrangement) and DβJβ-TRECs (Dβ1-Jβ1 rearrangements) (**Tables 1 and 2**), were used to obtain cloning vectors of known concentrations of sj-TRECs and DβJβ-TRECs. Two PCR reactions were carried out independently to amplified sj-TREC region, using 200 nM of primers (DTF6, sense; DTR61, antisense; see **Table 1**), and six fragments of DβJβ-TRECs, using 80 nM of sense primers (T3A to T3F see **Table 1**) and 280 nM of antisense primer (AO5AS; see **Table 1**). Both first rounds were performed in triplicates using KAPA HiFi DNA polymerase (Roche).

Subsequently, the amplification products of sj-TREC and DβJβ-TRECs were cloned into the pGEM T-EASY vector according to the manufacturer's instructions (Promega). The concentration of the plasmid was determined by quantitative real-time PCR using SP6/T7 primers (SP6/T7 transcription kit, Merck) and LightCycler® FastStart DNA MasterPLUS SYBR Green I (Roche Molecular Biochemicals). The standard curve was obtained by mixing the sj-TREC and DβJβ-TRECs (Jβ1.1, Jβ1.3, Jβ1.4 and Jβ1.6) plasmid at a ratio of 25/1 (sj-TREC/DβJβ-TREC), as disclosed by Fernando-Martinez S. *et al* (Ferrando-Martinez S, et al.; J Immunol Methods. 2010;352(1-2):111-117). Different concentrations were obtained through serial dilutions of a standard curve containing known concentrations of the first amplification reaction product of both regions (sj-TREC, from 1500 to 0 copies/μL; DβJβ-TRECs, from 50 to 0 copies/μL).

*sj/DβJβ-TREC ratio measurement by droplet digital PCR (ddPCR)*

sj-TREC and DβJβ-TRECs were quantified from extracted DNA (≥ 30 ng/ μL) by ddPCR. The sj-TREC and DβJβ-TRECs ddPCR mix per reaction was composed of 4 μL of DNA, 10 μL of ddPCR Supermix for probes no dUTP (Bio-Rad), 870nM of T2, R66, and F7 primers (see **Table 3**), 87 nM of T3A, T3B, T3C, T3D, T3E and T3F primers (see **Table 3**), 260 nM of each probe and water until reaching a final volume of 23 μL. Rribonuclease P/MRP subunit p30 (RPP30) was used as a housekeeping gene (GenBank accession number NM_001 104546.2). RPP30 mix was prepared with the same conditions of sj-TREC and DβJβ-TRECs mix, 4 μL of DNA ,10 μL of of ddPCR Supermix for probes no dUTP (Bio-Rad) and 23 μL of final volume. Primers were used at 870 nM and probe at 260 nM for RPP30 mix. All samples were assayed in duplicate. Primers and probes used for the detection of sj-TREC, DβJβ-TRECs and RPP30 are shown in **Table 3.** sj-TREC and DβJβ-TRECs primers were previously used in the qPCR.

**Table 3. Specific primers for the quantification of sj-TREC, DβJβ-TRECs and RPP30 by ddPCR.** The sequence direction is from the 5' to the 3' end.

| sj-TREC primers | Sequence (5'→3') | SEQ ID NO: |
|---|---|---|
| DTF7 | AGGCTCTGTCTAGTGTGATAAC | 10 |
| DTR66 | TGACATGGAGGGCTGAAC | 11 |
| **DβJβ-TRECs primers** | Sequence (5'→3') | SEQ ID NO: |
| T3A | CTTTCGATGGACCCTCACAG | 20 |
| T3B | GACAAGGCACCAGACTCACAG | 21 |
| T3C | AAGCTCTGGAAGGGAACACAG | 22 |
| T3D | CCGTTTCTCTCCCTCACACAG | 23 |
| T3E | GGGCAGAAACTGAGAACACAG | 24 |
| T3F | CTTGCGCCTTATGCTGCACAG | 25 |
| T2 | CCCAGGAGGAAAGAAGAGGAC | 13 |
| **sj-TREC probe** | Sequence (5'→3') | SEQ ID NO: |
| SD1 | CACCCCTCTGTTCCCCACA | 26 |
| **DβJβ-TREC probe** | Sequence (5'→3') | SEQ ID NO: |
| PB1 | TGGGAGTTGGGACCGCCAGAGAGG | 27 |
| **RPP30 primers** | Sequence (5'→3') | SEQ ID NO: |
| Forward | GATTTGGACCTGCGAGCG | 28 |
| Reverse | GCGGCTGTCTCCACAAGT | 29 |
| **RPP30 probe** | Sequence (5'→3') | SEQ ID NO: |
| RPP30 | CTGACCTGAAGGCTCT | 30 |

The probes have a detectable moiety, thus the SD1probe of SEQ ID NO: 26 is labeled at the 5'end with the fluorophore HEX and at the 3'end with the fluorescent quencher BHQ1. The PB1 probe of SEQ ID NO: 27 is bound at the 5' end with the fluorophore 6FAM and at the 3' end with the fluorescent quencher BHQ1. The RPP30 probe of SEQ ID NO: 30 is labeled at the 5'end with the fluorophore VIC and at the 3'end with the fluorescent quencher BHQ1.

ddPCR equipment comprises Bio-Rad QX200 droplet generator, PCR thermal cycler and Bio-Rad droplet digital reader (**Figure 1B.I**, **B.II and B.III**). First, the samples were separated into independent partitions with one, more, or none target by the Bio-Rad QX200 droplet generator **(Figure 1B.I).** Then, the amplification was carried out by a PCR thermal cycler (Bio-Rad) **(Figure 1B.II).** The following protocol consisted of a step for the enzyme activation at 95°C for 10 min, followed by 40 cycles of 30 see at 94°C of denaturation and 1 min at 59°C of annealing, and finally an enzyme deactivation of 10 min at 98°C. Finally, the quantification of sj-TREC and DβJβ-TRECs was determined by Bio-Rad droplet digital reader (Bio-Rad) **(Figure 1B.III)**. The data was represented as follows:

$$N^{\underline{o}} \; cells \; per \; million \; of \; \text{D}\beta\text{J}\beta \; or \; sj \; TRECs = \left( \dfrac{\dfrac{N^{\underline{o}} \text{ copies of } \text{D}\beta\text{J}\beta \text{ or } sj * 1{,}000{,}000}{N^{\underline{o}} \text{ Copies of Housekeeping gene (i. e. RPP30)}}{2} \right)$$

The ratio was calculated based on the known concentrations of sj-TREC divided by DβJβ-TRECs.

*Statistical Analysis*

Nonparametric statistical analysis was performed using Statistical Package for the Social Sciences software (SPSS 22.0; SPSS, Inc.), and graphs were done using GraphPad Prism version 8.4.2 (GraphPad software, Inc). Illustrations were created using the BioRender web server. Median and interquartile ranges were used to describe continuous variables and percentages to describe categorical variables. Correlations between variables were assessed using Spearman's rank test. All *p* values <0.05 were considered significant. The number of copies of both regions was determined using Bio-Rad QuantaSoft software v.1.7.4.

**Example 1. Detection limit of sj/DβJβ-TREC ratio by ddPCR**

A standard curve was designed with the previously obtained plasmids from the clones of DβJβ-TREC and sj-TREC regions (see materials and methods section). To determine the efficacy and detection limit of the method of the present disclosure, different concentrations of clones of DβJβ-TREC and sj-TREC regions were measured through a 10-fold serial dilution of a standard curve of known concentrations for determining the sj/β-TREC ratio. The standard curve was performed in triplicates **(Figure 2).**

Using this approach, the detection limit was 2 copies/μL (1.93±0.50 for sj-TREC and DβJβ-TREC 0.03±0.00 copies/μL, respectively) for sj-TREC and DβJβ-TREC levels (0.6 ± 0.20 and 0.15 ± 0.061 copies, respectively) **(Table 4).** These results demonstrated that ddPCR determination is a reliable and accurate method to use for the determination of thymic function.

**Table 4. Number of sj-TREC and DβJβ-TREC copies at different concentrations of cloning vectors, through** a **10-fold serial dilution.** Data is represented as the average ± standard deviation number of copies of three standard curves run in triplicates and independent experiments.

| Dilutions | sj-TREC copies | DβJβ-TREC copies |
|---|---|---|
| Dilution 1 | 1586 ±139.69 | 43.57t0.55 |
| Dilution 2 | 137.27±40.42 | 3.80±1.41 |
| Dilution 3 | 15.70±2.52 | 0.46±0.15 |
| Dilution 4 | 1.93±0.50 | 0.03±0.00 |
| Dilution 5 | 0.20±0.05 | 0.00±0.00 |

**Example 2. Comparison of sj/DβJβ-TREC ratio levels in elderly healthy donors by qPCR and ddPCR.**

Thymic function has a pivotal role in chronological aging. Thymic function persists in adulthood up to 107 years of age and failures in this function are associated with mortality in elderly population. Thus, we compared the sj/DβJβ-TREC ratio in 26 elderly subjects with a median age of 76 [IQR, (70.5-87)] years old using the method of the present disclosure, row data of a participant is shown in **Figure 3,** and the previous method, ddPCR and qPCR, respectively.

While significant correlations were observed between the previous method (qPCR) and ddPCR for sj-TREC levels (p<0.004, r=0.548) **(Figure 4A)** and sj/DβJβ-TREC ratio (p=0.033, r=0.418) **(Figure 4B)**, no differences were observed for DβJβ-TREC levels (p=0.147, r=-0.292) **(Figure 4C)**. Interestingly, using the qPCR method, 28% of DβJβ-TREC measurements were below the detection limit while 96.15% were detectable by ddPCR Not significant associations were found between the age of the participants and the thymic function analysed by sj-TREC (p=0.068 r=-0.363) and DβJβ-TREC (p=0.111 r=0.588) parameters. Nevertheless, a significant inverse correlation was found between the age of the participants and the thymic function analysed by the sj/DβJβ-TREC ratio parameter with the qPCR (p=0.009 r=-0.501) **(Figure 5A)** and ddPCR method (p=0.016 r=-0.468) **(Figure 5B).** This result highlights the higher sensitivity and precision of ddPCR to detect the DβJβ-TRECs region in comparison with conventional qPCR.

**Example 3. Comparison of sj/DβJβ-TREC ratio levels in people living with HIV by qPCR and ddPCR.**

It is known that thymic function measurement is also important in lymphopenic scenarios such as HIV-disease progression. Indeed, the thymus has an important role in the immune regeneration that takes place in HIV infection after antiretroviral therapy or cancer after chemotherapy. In the HIV scenario, the thymic function contributes to the maintenance of CD4 T-cell levels in different PLHIV immunological phenotypes.

In this sense, the sj/DβJβ-TREC ratio of 54 PLHIV with a median age of 32.5 ([13.75-42.25]) years old, classified as typical progressors, LTNPs, and vertically HIV-infected subjects was measured by ddPCR according to the method of the present disclosure, and qPCR methods. Significant positive correlations between both techniques were observed for sj-TREC levels (p<0.0001, r=0.736) (**Figure 6A**), sj/DβJβ-TREC ratio (p<0.003, r=0.403) (**Figure 6B**) and DβJβ-TREC levels (p=0.001, r=0.443) (**Figure 6C**). Furthermore, significant inverse correlations were found between the age of the subjects and sj-TREC levels (p<0.0001, r=-0.712) (**Figure 7A**) and sj/DβJβ-TREC ratio (p<0.0001, r=-0.712) (**Figure 7B**) but not in DβJβ-TREC levels (p=0.209, r=-0.174) **(Figure 7C).** Therefore, the thymic function is an important parameter and its detection by ddPCR becomes a powerful tool in early diagnosis to categorize individuals with different progression rates.

Taken together, results presented herein show that the detection limit for the sj/DβJβ-TREC ratio by ddPCR, according to the method of the present disclosure, as < 2 copies demonstrates that ddPCR determination is a reliable and accurate method to use for the determination of thymic function. Moreover, a higher sensitivity and precision of ddPCR, according to the method of the present disclosure, to detect the DβJβ-TRECs region in comparison with conventional qPCR was demonstrated. Finally, a positive correlation of sj-TREC levels, DβJβ-TREC levels and sj/DβJβ-TREC ratio using both methods was observed. Therefore, the thymic function is an important parameter and its detection by the method disclosed herein becomes a powerful tool in early diagnosis and/or prognosis of diseases characterized by an immune system disfunction, such as, autoimmune disease, acute immunodeficiency syndrome, cancer, etc. Evaluation of immune therapy efficacy, such as vaccines including prophylactic vaccines on normal individuals, bone marrow transplantations, etc, can be performed by the methos of the present disclosure.

**Claims**

1.   Method *in vitro* and/or *ex vivo* for assessing and monitoring the thymic function in an isolated biological sample from an individual, wherein the method comprising:

   a) Measuring the expression levels of sj-TREC (SEQ ID NO:32) and DβJβ-TREC (SEQ ID NO: 31) in the isolated sample by ddPCR;
   b) Determining the sj/DβJβ-TREC ratio;
   c) Repeating steps (a) and (b) using the same type of biological sample but obtained from the individual at a subsequent point in time;
   d) Comparing the sj/DβJβ-TREC ratio measured in step (b) with that measured in step (c), thereby monitoring the progression or regression of thymic function in the individual.

2.   Method *in vitro* and/or *ex vivo* for the prognosis and/or diagnosis of an immune system dysfunction in an isolated biological sample from an individual, wherein the method comprising:

   a) Measuring the expression levels of sj-TREC (SEQ ID NO: 32) and DβJβ-TREC (SEQ ID NO: 31) in the isolated sample by ddPCR;
   b) Determining the sj/DβJβ-TREC ratio;

   wherein a value of said sj/DβJβ-TREC ratio is different from that found in a healthy individual of the same age, is an indication that the individual has an immune system dysfunction.

3.   Method *in vitro* and/or *ex vivo* for assessing the effect of a therapeutic treatment in an isolated biological sample from an individual, wherein the method comprising:

   a) Measuring the expression levels of sj-TREC (SEQ ID NO: 32) and DβJβ-TREC (SEQ ID NO: 31) in the isolated sample by ddPCR;
   b) Determining the sj/DβJβ-TREC ratio;
   c) Repeating steps (a) and (b) using the same type of biological sample but obtained from the individual at a subsequent point in time after to administering the therapeutic treatment;

d) Comparing the sj/DβJβ-TREC ratio measured in step (b) with that measured in step (c), thereby assessing an effect of the therapeutic treatment in the individual.

4. Method according to anyone of claims 1 to 3, wherein the primers used for measuring the expression level of sj-TREC are set for as SEQ ID NOs: 10 and 11, and the probe is set for as SEQ ID NO: 26; and the primers used for measuring the expression level of DβJβ-TREC are set forth as SEQ ID NOs: 13 and 20 to 25, and the probe is set for as SEQ ID NO: 27.

5. Method according to anyone of claims 1 to 4, wherein the isolated biological sample is a blood sample, preferably a whole blood sample and more preferably a sample of peripheral blood mononuclear cells.

6. Method according to anyone of claims 1 and 3-5, wherein said assessing and monitoring is for evaluating a therapy's efficacy; a therapy's side effects, the effect on thymic function of a disorder or disease and/or the effect on thymic function of a therapeutic treatment.

7. Method according to claim 6, wherein the therapy's efficacy and/or the effect on thymic function of a therapeutic treatment is assessed and monitored from a therapy selected from the group consisting of vaccination, antiviral therapy, T-cell reconstitutions, bone marrow transplantation and hematopoietic stem cell transplantation.

8. Method according to claim 6 or 7, wherein the therapy's side effects and/or the effect on thymic function of a therapeutic treatment are assessed and monitored from a therapy selected from the group consisting of radiotherapy, chemotherapy and drug therapy.

9. Method according to anyone of claims 1 to 8, wherein the individual is an immunocompromised individual having an immune system dysfunction.

10. Method according to anyone of claims 1 to 9 wherein the immune system dysfunction is selected from the list consisting of: acute and chronic viral infections, preferably a viral infection selected from human immunodeficiency virus or acquired immunodeficiency syndrome (HIV/AIDS), hepatitis C virus (HCV), Epstein-Barr virus (EBV); autoimmune diseases, preferably multiple sclerosis, Crohn's disease; autoimmune endocrine disorders, preferably autoimmune polyglandular syndromes, Graves' disease, immune mediated diabetes; rheumatoid arthritis, systemic lupus erythematosus; cancers, bone marrow transplantation (BMT), organ transplantation and allogeneic haematopoietic stem cell transplantation (AHSCT).

11. Use *in vitro* and/or *ex vivo* of the expression level of sj-TREC (SEQ ID NO: 32), DβJβ-TREC (SEQ ID NO: 31) and sj/β-TREC ratio, obtained by ddPCR, as biomarkers for:

   i. assessing and monitoring thymic function in an isolated biological sample from an individual;
   ii. prognosis and/or diagnosis of an immune system dysfunction in an isolated biological sample from an individual; and/or
   iii. assessing an effect of a therapeutic treatment in an isolated biological sample from an individual.

12. Use according to claim 11, wherein the primers and probes used for measuring the expression level of sj-TREC are set for as SEQ ID NOs: 10 and 11, and the probe is set for as SEQ ID NO: 26; and the primers used for measuring the expression level of DβJβ-TREC are set forth as SEQ ID NOs: 13 and 20 to 25, and the probe is set for as SEQ ID NO: 27.

13. Kit for assessing and monitoring thymic function, for the prognosis and/or diagnosis of an immune system dysfunction and/or for assessing an effect of a therapeutic treatment, in an isolated biological sample from an individual, wherein the kit comprises reagents for amplifying the sj-TREC (SEQ ID NO: 32) and DβJβ-TREC (SEQ ID NO: 31) in said sample by ddPCR.

14. Kit according to claim 13, wherein the primers and probes for amplifying the sj-TREC (SEQ ID NO: 32) and DβJβ-TREC (SEQ ID NO: 31) are set for as SEQ ID NOs: 10 and 11 as primers, and SEQ ID NO: 26 as the probe for the sj-TREC; and SEQ ID NOs: 13 and 20 to 25 as primers and SEQ ID NO: 27 as probe for the DβJβ-TREC.

**FIGURE 1**

FIGURE 2

FIGURE 3A

FIGURE 3B

FIGURE 4A

FIGURE 4B

FIGURE 4C

FIGURE 5A

**FIGURE 5B**

**FIGURE 6A**

**FIGURE 6B**

**FIGURE 6C**

**FIGURE 7A**

**FIGURE 7B**

FIGURE 7C

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BULNES-RAMOS ÁNGEL ET AL: "Factors associated with the humoral response after three doses of COVID-19 vaccination in kidney transplant recipients", FRONTIERS IN IMMUNOLOGY, vol. 14, 18 February 2023 (2023-02-18), XP93141158, Lausanne, CH ISSN: 1664-3224, DOI: 10.3389/fimmu.2023.1099079 Retrieved from the Internet: URL:https://www.ncbi.nlm.nih.gov/pmc/articles/doi/10.3389/fimmu.2023.1099079> | 1-3, 5-11,13 | INV. C12Q1/6883 C12Q1/606 |
| Y | * the whole document * * p. 2, col. 2, first para., p. 3, col. 1, third para., p. 3, col. 2, fourth para., p. 4, col. 2, fourth para., p. 4, col. 2, seventh para., p. 4, col. 2, first para., p. 5, Table 2, p. 8, col. 2, first para., Supplementary Table 1 * | 4,12,14 | |
| | ----- | | TECHNICAL FIELDS SEARCHED (IPC) |
| X | WO 2011/086161 A2 (UNIV NOTRE DAME DE LA PAIX [BE]; UNIV LIEGE [BE] ET AL.) 21 July 2011 (2011-07-21) | 13,14 | C12Q |
| A | * the whole document * * para. 25, 26, 40-41, 139; claims 21-22 * | 1-12 | |
| | ----- | | |
| Y | EP 2 284 280 A1 (FUNDACION REINA MERCEDES PARA LA INVESTIGACION SANITARIA [ES] ET AL.) 16 February 2011 (2011-02-16) * the whole document * * para. 2, 13, 17, figures 1, 5, 9. * | 4,12,14 | |
| | ----- | | |
| | -/-- | | |

The present search report has been drawn up for all claims

| Place of search | Date of completion of the search | Examiner |
|---|---|---|
| Munich | 2 April 2024 | Sauer, Tincuta |

EPO FORM 1503 03.82 (P04C01)

page 1 of 3

| | Europäisches Patentamt<br>European Patent Office<br>Office européen des brevets | **EUROPEAN SEARCH REPORT** | **Application Number**<br>**EP 23 38 3145** |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FERRANDO-MARTINEZ S ET AL: "A reliable and simplified sj/beta-TREC ratio quantification method for human thymic output measurement",<br>JOURNAL OF IMMUNOLOGICAL METHODS, ELSEVIER SCIENCE PUBLISHERS B.V.,AMSTERDAM, NL,<br>vol. 352, no. 1-2,<br>31 January 2010 (2010-01-31), pages 111-117, XP026808314,<br>ISSN: 0022-1759<br>[retrieved on 2009-11-15]<br>* the whole document *<br>* p. 113, col. 2, second para., p. 115, col. 1, second para., p. 115, col. 2, second para. * | 1-14 | |
| Y | TESSITORE MARION VAGLIO ET AL: "Detection of newly produced T and B lymphocytes by digital PCR in blood stored dry on nylon flocked swabs",<br>JOURNAL OF TRANSLATIONAL MEDICINE,<br>vol. 15, no. 1,<br>1 December 2017 (2017-12-01), XP093141397,<br>ISSN: 1479-5876, DOI:<br>10.1186/s12967-017-1169-9<br>* the whole document *<br>* p. 4, col. 1, second para., p. 6, col. 2, third para., p. 8, col. 1, second para. * | 1-14 | TECHNICAL FIELDS SEARCHED (IPC) |

-/--

The present search report has been drawn up for all claims

| Place of search | Date of completion of the search | Examiner |
|---|---|---|
| Munich | 2 April 2024 | Sauer, Tincuta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 23 38 3145

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PROFAIZER TRACIE ET AL: "A Multiplex, Droplet Digital PCR Assay for the Detection of T-Cell Receptor Excision Circles and Kappa-Deleting Recombination Excision Circles", CLINICAL CHEMISTRY, vol. 66, no. 1, 1 January 2020 (2020-01-01), pages 229-238, XP93139747, US ISSN: 0009-9147, DOI: 10.1373/clinchem.2019.308171 Retrieved from the Internet: URL:http://academic.oup.com/clinchem/article-pdf/66/1/229/31700786/clinchem.2019.308171.pdf> * the whole document * * p. 230, col. 1, first para., p. 237, col. 1, third para. * | 1-14 | |
| Y | Vitallé Joana ET AL: "Immune defects associated with lower SARS-CoV-2 BNT162b2 mRNA vaccine response in aged people", JCI insight, 8 September 2022 (2022-09-08), pages 1-20, XP93140973, United States DOI: 10.1172/jci.insight.161045 Retrieved from the Internet: URL:https://www.ncbi.nlm.nih.gov/pmc/articles/PMC9536264/pdf/jciinsight-7-161045.pdf [retrieved on 2024-03-13] * the whole document * * p. 3, col. 1, second para., p. 17, col. 1, third para. * | 1-14 | TECHNICAL FIELDS SEARCHED (IPC) |

The present search report has been drawn up for all claims

| Place of search | Date of completion of the search | Examiner |
|---|---|---|
| Munich | 2 April 2024 | Sauer, Tincuta |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

**EP 23 38 3145**

This annex lists the patent family members relating to the patent documents cited in the above-mentioned European search report.
The members are as contained in the European Patent Office EDP file on
The European Patent Office is in no way liable for these particulars which are merely given for the purpose of information.

**02-04-2024**

| Patent document cited in search report | Publication date | Patent family member(s) | Publication date |
|---|---|---|---|
| WO 2011086161 A2 | 21-07-2011 | US 2013288255 A1 | 31-10-2013 |
| | | WO 2011086161 A2 | 21-07-2011 |
| EP 2284280 A1 | 16-02-2011 | EP 2284280 A1 | 16-02-2011 |
| | | ES 2331499 A1 | 05-01-2010 |
| | | US 2011287954 A1 | 24-11-2011 |
| | | WO 2009027568 A1 | 05-03-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4458066 A
- US 4415732 A
- US 4914210 A, Levenson

### Non-patent literature cited in the description

- **FERRANDO-MARTINEZ S et al.** *J Immunol Methods*, 2010, vol. 352 (1), 111-117
- **DION M-L et al.** *Methods Mol Biol*, 2007, 197-213
- **HARRIS JM et al.** *Clin Immunol.*, 2005, vol. 115 (2), 138-146
- **HAZENBERG MD et al.** *Nat Med.*, 2000, vol. 6 (9), 1036-1042
- **DION M-L et al.** *Methods Mol Biol.*, 2007, 197-213
- **DEN BRABER I et al.** *Immunity.*, 2012, vol. 36 (2), 288-297
- **DION M-L et al.** *Immunity.*, 2004, vol. 21, 757-768
- **FERRANDO-MARTINEZ S et al.** *J Immunol Methods.*, 2010, vol. 352 (1), 111-117
- **HUGGETT et al.** *Clinical Chemistry*, 2013, vol. 59, 1691-1693
- **SHUGA et al.** *Nucleic Acids Research*, 2013, vol. 41 (16), el59
- **WHALE et al.** *PLoS One*, 2013, vol. 3, e58177
- **BAO et al.** *Annual Review of Biomedical Engineering*, 2009, vol. 11, 25-47
- **OZAKI et al.** *Nucleic Acids Research*, 1992, vol. 20, 5205-5214
- **AGRAWAL et al.** *Nucleic Acids Research*, 1990, vol. 18, 5419-5423
- **BEAUCAGE et al.** *Tetrahedron*, 1992, vol. 48, 2223-2311
- *Applied Biosystems User Bulletin*, 01 April 1987 (13)
- **NARANG et al.** *Meth. Enzymol.*, 1979, vol. 68, 90
- **BROWN et al.** *Meth. Enzymol.*, 1979, vol. 68, 109
- **CLOAD et al.** *J. Am. Chem. Soc.*, 1991, vol. 113, 6324-6326
- **DURAND et al.** *Nucleic Acids Res.*, 1990, vol. 18, 6353-6359
- **HORN et al.** *Tet. Lett.*, 1986, vol. 27, 4705-4708
- **GARCIA-MERINO I et al.** *Retrovirology.*, 2009, vol. 6, 1-5